# EUROPEAN PATENT APPLICATION

(11) **EP 2 842 558 A1**
(43) Date of publication of application: **04.03.2015**
(21) Application number: 13781857.1
(22) Date of filing: 23.04.2013
(51) Int. Cl.: A61K 31/365, A61P 3/00, A61P 9/00, A61P 17/00, A61P 17/02, A61P 43/00, C07D 493/04

(54) **CGRP RESPONSIVENESS PROMOTER**

(30) Priority: 24.04.2012 JP 2012098963
(71) Applicant: Kao Corporation, Chuo-ku Tokyo 103-8210 (JP)
(72) Inventor: NOMURA, Tomoko, Haga-gun Tochigi 3213497 (JP); ICHINOSE, Susumu, Haga-gun Tochigi 321-3497 (JP)
(74) Representative: Vossius & Partner
(86) International application number: PCT/JP2013/061932
(87) International publication number: WO 2013/161821

(57) **Abstract**

A material capable of promoting cellular responsiveness to CGRP is provided. A lignan represented by the following formula (I), wherein in formula (I), R₁ represents a hydrogen atom or an alkoxy group; and R₂, R₃ and R₄, which may be identical or different, each represent an alkoxy group, is used for the promotion of cellular responsiveness to CGRP,

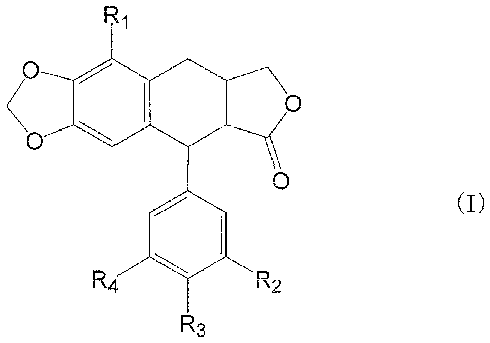

## Description

### Field of the Invention

The present invention relates to a CGRP (calcitonin gene-related peptide) responsiveness promoter.

### Background of the Invention

In the skin, there is formed a vascular plexus composed of three layers of vascular beds that are parallel to the skin surface. This vascular plexus contributes to skin circulation. One of the main functions of the skin circulation is body temperature regulation, and when a cold stimulus or a heat stimulus is applied, the skin blood vessels contract or expand and relax, thereby causing a change in the blood flow of the skin circulation, and the body temperature is maintained by suppressing or promoting body heat radiation. Skin circulation also has a key role in terms of regulation of the distribution of systemic blood flow, and is affected by not only information from the central or peripheral caloreceptors, but also by information from baroreceptors, volume receptors or chemoreceptors, or the vasomotor reflex caused by motion.

Skin circulation undergoes changes caused with aging. It is known that along with aging, the structure of the skin vascular plexus or the amount of blood flow is changed, and the amount of change in blood flow due to a cold stimulus or a heat stimulus isdecreased. Consequently, there may occur a problem that blood circulation of the skin is deteriorated along with aging, and thereby the metabolism of the skin is further reduced.

Skin circulation is regulated two-dimensionally by systemic mechanisms involving the sympathetic nervous system and local mechanisms involving local regulators. Axon reflux is one of the local mechanisms. This is a phenomenon in which the impulse generated in the sensory nerves of the skin by stimulation is transferred retrogradely to other branches in the process of conducting the impulse to the center, the impulse releases a neuropeptide from the nerve endings thereof, and the skin blood vessels expand. These neuropeptides are substance P (SP) or calcitonin gene-related peptide (CGRP).

CGRP is a neuropeptide composed of 37 amino acids, and is biosynthesized by tissue-specific selective splicing of calcitonin gene. CGRP is widely distributed in the central nervous system and the sensory peripheral nervous system, and exhibits its actions through specific receptors. It is well known that CGRP receptors are expressed in vascular endothelial cells and vascular smooth muscle cells. In either of the cells, when CGRP is bound to a receptor therein, the blood vessels are dilated, and the blood flow is increased. In vascular endothelial cells, when CGRP is bound to a CGRP receptor of a cell, intracellular cAMP is increased as a result of G protein-mediated activation of adenylate cyclase. The increase in cAMP causes activation of protein kinase A, followed by activation of eNOS, and thereby production of NO is promoted. The NO thus produced acts on vascular smooth muscle cells, and K⁺ channel is opened via NO-mediated activation of intracellular cGMP. Thereby, the vascular smooth muscles are relaxed, blood vessels are dilated, and consequently, the blood flow is increased. In a case in which CGRP is bound directly to the vascular smooth muscle cells, G protein-mediated activation of an adenylate cyclase induces the activation of protein kinase A, and the K⁺ channel is opened. As a result, the vascular smooth muscles are relaxed as well, blood vessels are dilated, and the blood flow is increased.

The relationship between CGRP and the blood flow is well known. For example, it is known that administration of CGRP in a rat increases the skin blood flow (Non-Patent Literature 1); the blood pressure of a CGRP knock-out mouse is increased (Non-Patent Literature 2); skin blood flow is decreased in a rat administered with a neutralizing antibody to CGRP (Non-Patent Literature 3); patients suffered from Raynaud's disease, which is considered to be caused by hypercontraction of peripheral blood vessels, lack the CGRP-containing nerves in fingers (Non-Patent Literature 4) ; when CGRP is intravenously injected to a patient with Raynaud's disease, the amount of skin blood flow in the arm is increased (Non-Patent Literature 5) and the like.

Physiological actions of CGRP include, in addition to the blood vessel dilating action, many actions such as regulation of substance P in inflammation, enhancement of vascular permeability, regulation of nicotine-like receptors at the neuromuscular junctions, suppression of glycogenesis and promotion of glycolysis, promotion of the secretion of pancreatic enzymes, suppression of the secretion of gastric acid, heartbeat promotion, regulation of neural activity, regulation of calcium metabolism, promotion of osteogenesis, insulin secretion, body temperature rise, and a drop in food intake. Furthermore, it is known that CGRP is also involved in an anti-inflammatory action mediated by prostaglandin synthesis, preconditioning for ischemic reperfusion disorder, and gestation and childbirth since receptors are expressed in the myometrium, uterus and placenta.

In view of the above-described physiological actions, examples of the use of CGRP or a CGRP-responsiveness promoter include promotion of blood circulation, promotion of blood flow, promotion of wound healing, and treatments for hypertension, cardiac failure, hepatic disorder caused by is chemic reperfusion, renal failure, gastrointestinal ulceration, septicemia, osteoporosis, arrhythmia, subarachnoid hemorrhage, pulmonary hypertension, delayed type hypersensitivity, gestational toxicosis and premature labor and the like. Furthermore, examples of the use of a CGRP receptor antagonist include treatments for migraine, hyperesthesia and the like. In fact, many CGRP receptor antagonist compounds have been developed as antimigraine drugs.

Furthermore, it is also known that there is a relation between CGRP and periodontal diseases. It has been reported in Non-Patent Literature 6 that on the occasion of inflammation, a large number of sensory nerves expressing CGRP enter, and CGRP exhibits anti-inflammation through suppression of H₂O₂ production in a macrophage, suppression of antigen presentation, or suppression of the division of lymphocytes. Also, it has been reported that since CGRP has a human pulpal cell proliferation promoting activity and is expected to exhibit dentinogenesis mediated by BMP-2 expression promotion, CGRP is related to dental pulp formation (Non-Patent Literatures 7 and 8). Furthermore, since CGRP has a bone resorption inhibitory action (Non-Patent Literature 9), examples of the therapeutic use of CGRP or a CGRP-responsiveness promoter also include periodontal diseases.

A substance capable of promoting or suppressing the responsiveness of skin circulation to CGRP is useful for an improvement of the change in skin circulation caused with aging as described above, or for the prevention or treatment of the diseases or symptoms described above. Substances capable of suppressing the responsiveness to CGRP, which are known hitherto, include a CGRP receptor antagonist, an anti-CGRP antibody, and an aqueous extract of iris (Patent Literature 1). However, it is still desired to develop another substance which can promote or suppress the responsiveness to CGRP, and is useful for the regulation of skin circulation or the amelioration of various diseases.

In regard to certain kinds of lignans, it has been hitherto disclosed that the lignans have actions of suppression of cellular adhesion, suppression of cancer metastasis and prevention and treatment of arteriosclerosis (Patent Literature 2); actions of suppression of NF-κB activation, regulation of gene expression, anti-human immunodeficiency virus, prevention or amelioration of inflammation, prevention or amelioration of adult diseases and suppression of cancer metastasis (Patent Literature 3); and actions of prevention or treatment of periodontal diseases (Patent Literature 4). Furthermore, it has been hitherto reported that podophyllotoxins, which are a kind of lignans, are cytotoxic and have activities such as a hepatic disorder suppressive activity, a 5-lipoxygenase inhibitory activity, an action of suppressing Ca inflow into cells caused by stimulation, an action of double inhibiting COX-2 and 5-lipoxygenase, an anti-angiogenic action, an antitumor activity, an antioxidizing action and an antiviral activity (Non-Patent Literatures 10 to 18).

Podophyllotoxins are known to be contained in a culture productofLinumflavumroots, *Anthriscus sylvestris* (cowparsley, wild chervil (*shaku*, *yamaninjin*))*, Pulsatilla koreana* (Korean pasque flower (*chosen okinagusa*))*,* or Hernandia sp. or *Hernandia nymphaeifolia* of the genus Hernandia of the Hernandiaceae family (Non-Patent Literatures 10, 19 and 20). Non-Patent Literatures 21 and 22 report on the blood vessel dilating action or antihypertensive action of plants of the genus Hernandia or components contained therein; however, the effectiveness of the plants is not necessarily clearly known. Furthermore, since plants of the genus Hernandia also contain other components in addition to podophyllotoxins, the contribution of podophyllotoxins on the efficacy of the plants of the genus Hernandia is not obvious.

### Citation List

### Patent Literature

Patent Literature 1: Japanese Patent No. 3093154
Patent Literature 2: JP 10-067656 A
Patent Literature 3: JP 10-175861 A
Patent Literature 4: JP 2005-162653 A

### Non Patent Literature

Non-Patent Literature 1: Nature 1988, 335:164-167
Non-Patent Literature 2: Hypertension, 2000, 35:470-475
Non-Patent Literature 3: Br J Pharmacol, 2008, 155:1093-1103
Non-Patent Literature 4: Lancet, 1990, 336:1530-1533
Non-Patent Literature 5: Lancet, 1989, 2(8676):1354-1357
Non-Patent Literature 6: Journal of Endodontics, 2008, 34(7):773-788
Non-Patent Literature 7: J Dent Res, 1995, 74:1066-1071
Non-Patent Literature 8: J Endod, 1997, 23:485-489
Non-Patent Literature 9: Calcif Tissue Int, 1987, 40:149-154
Non-Patent Literature 10: Journal of Natural Products, 1986, 49(3):435-439
Non-Patent Literature 11: Yakugaku Zasshi, 1985, Volume 105(2):109-118
Non-Patent Literature 12: Planta Medica, 2007, 73 (13) :1331-1357
Non-Patent Literature 13: Archives of Toxicology, 2002, 75(11):695-702
Non-Patent Literature 14: Biol Pharm Bull, 2004, 27:786-788
Non-Patent Literature 15: Planta Medica, 2002, 68:271-274
Non-Patent Literature 16: Bioorganic and Medicinal Chemistry Letters, 2009, 19(15):4367-4371
Non-Patent Literature 17: Acta Horticulturae, 2003, 597: 191-198
Non-Patent Literature 18: Archiv der Pharmazie, 1994, 327(3):175-179
Non-Patent Literature 19: Bioorganic and Medicinal Chemistr, 2005, Volume 13(21):5892-5908
Non-Patent Literature 20: Planta Medica, 1996, 62:528-533
Non-Patent Literature 21: Planta Medica, 2001, 67:593-598
Non-Patent Literature 22: Records of Natural Products, 2009, 3(1):1-22

### Summary of the Invention

The present invention provides use of a lignan represented by the following formula (I), for the production of a cellular CGRP-responsiveness promoter, a skin circulation improver, a blood circulation or blood flow promoter, a skin metabolism improver, or a wound healing promoter: 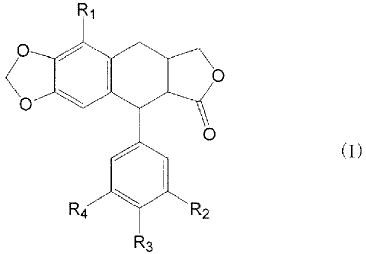 wherein R₁ represents a hydrogen atom or an alkoxy group; and R₂, R₃ and R₄, which may be identical or different, each represent an alkoxy group.

Furthermore, the present invention provides use of a lignan represented by the above formula (I), for the promotion of cellular responsiveness to CGRP, improvement of skin circulation, promotion of blood circulation or blood flow, improvement of skin metabolism or promotion of wound healing.

Furthermore, the present invention provides a lignan represented by the above formula (I), for use in the promotion of cellular responsiveness to CGRP, improvement of skin circulation, promotion of blood circulation or blood flow, improvement of skin metabolism or promotion of wound healing.

Furthermore, the present invention provides a method for promoting cellular responsiveness to CGRP, improving skin circulation, promoting blood circulation or blood flow, improving skin metabolism, or promoting wound healing in a human being or a non-human mammal, the method comprising administering a lignan represented by the above formula (I) to the human being or the non-human mammal.

### Brief Description of Drawings

Fig. 1 illustrate the promotion of CGRP-responsiveness of HCASMC, wherein A illustrates the compound TD-1 and B illustrates the compound TD-2. *: p < 0.05, **: p < 0.01 (vs. control, Dunnett's test, N = 3).
Fig. 2 illustrates the promotion of CGRP-responsiveness of HCASMC by the compounds TD-1 and TD-2 added under different conditions. *: p < 0.05, **: p < 0.01 (vs. control, Dunnett's test, N = 3).

### Detailed Description of the Invention

In the present specification, the term "non-therapeutic" is a concept that does not include medical practice, that is, the practice of remedy performed on a human body by a treatment. It is a concept.

In the present specification, the term "improvement" refers to amelioration of a disease or a symptom, prevention or delay of the aggravation of a disease or a symptom, or reversal, prevention or delay of the progress of a disease or a symptom.

In the present specification, the term "prevention" refers to prevention or delay of the onset of a disease or a symptom in an individual, or lowering of the risk of the onset of a disease or a symptom of an individual.

In the present specification, "promotion of cellular responsiveness to CGRP" means promotion of any cellular activity caused by CGRP. When CGRP is bound to a CGRP receptor of a cell, cAMP in the cell is increased through the G protein-mediated activation of adenylate cyclase. As a result of the increase incAMP, for example, in vascular smooth muscle cells, a K⁺ channel is opened by means of protein kinase A activation. As a result, vascular smooth muscle cells are relaxed, blood vessels are dilated, and the blood flow is increased. Or else, in vascular endothelia cells, eNOS is activated through protein kinase A activation, and production of NO is promoted. Furthermore, the NO thus produced acts on the vascular smooth muscle cells, and a K⁺ channel is opened via NO-mediated cGMP activation in the cells. As the result, the vascular smooth muscle cells are relaxed, blood vessels are dilated, and the blood flow is increased. A cellular CGRP-responsiveness promoter according to the present invention can promote a series of these processes in cells that are caused by CGRP. Examples of the actions of the CGRP responsive promoter of the present invention include promotion of binding between CGRP and a receptor thereof, enhancement of the G protein activity, enhancement of the adenylate cyclase activity, increase in intracellular cAMP, enhancement of the protein kinase A activity, enhancement of NO production or cGMP activity, and promotion of K⁺ channel opening. Preferably, the "cellular responsiveness to CGRP" can be determined by using the degree of increase in intracellular cAMP caused by CGRP as an index. Therefore, the CGRP-responsiveness promoter of the present invention preferably promotes an increase in intracellular cAMP caused by CGRP.

The "cell" for which CGRP-responsiveness is promoted in the present invention is not particularly limited as long as it is a cell which expresses a CGRP receptor, or a cell having a CGRP receptor. Preferred examples of the cell include a vascular smooth muscle cell, a vascular endothelial cell, a fibroblast, an osteoblast, an ameloblast and an odontoblast, and more preferred examples include a vascular smooth muscle cell and a vascular endothelial cell. Alternatively,the"cell" may be a cell debris or a cell fraction of any of the cells listed above, or may also be a tissue containing any of the cells listed above, or a culture product derived from any of the cells listed above.

The present invention relates to a provision of a cellular CGRP-responsiveness promoter.

The inventors of the present invention conducted an investigation on a material that is capable of promoting cellular responsiveness to CGRP, and found that a lignan having a particular structure has a CGRP-responsiveness promoting action.

The present invention provides a CGRP-responsiveness promoter that is capable of promoting cellular responsiveness to CGRP. The CGRP-responsiveness promoter is useful for the amelioration of a change in skin circulation caused by, for example, aging, or for the prevention or treatment of various diseases or symptoms associated with CGRP.

The cellular CGRP-responsiveness promoter according to the present invention comprises a lignan represented by the following formula (I) as an active ingredient:

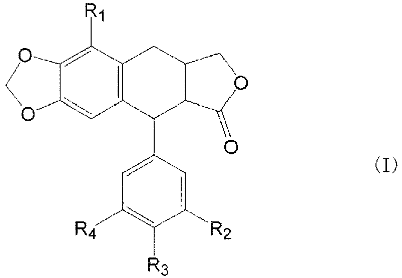

In the formula (I), R₁ represents a hydrogen atom or an alkoxy group, and R₂, R₃ and R₄, which may be identical or different, each represent an alkoxy group. The alkoxy group for R₁ to R₄ is preferably a linear or branched group having 1 to 10 carbon atoms, and examples thereof include a methoxy group, an ethoxy group, an n-propyloxy group, an isopropyloxy group, an n-butyloxy group, a sec-butyloxy group, a tert-butyloxy group, a pentyloxy group, a hexyloxy group, a heptyloxy group, an octyloxy group, a nonyloxy group, a decyloxy group and the like. The alkoxy group is preferably an alkoxy group having 1 to 3 carbon atoms, more preferably a methoxy group.

The lignan represented by the above formula (I) (in the present specification, hereinafter, referred to as lignan of formula (I)) can be extracted from, for example, *Thujopsis dolabrata* (mainly leaves and twigs). Extraction is carried out by subjecting *Thujopsis dolabrata* or a dried powder thereof to an extraction treatment usually at 3°C to 70°C using water, an organic solvent (for example, petroleum ether, n-hexane, cyclohexane, carbon tetrachloride, toluene, benzene, dichloromethane, chloroform, ether, ethyl acetate, butanol, acetone, propanol, ethanol, methanol, pyridine, polyethylene glycol, propylene glycol, butylene glycol or the like), or a mixture thereof (for example, an aqueous alcohol).

A preferred example of extraction of the lignan of formula (I) from a raw material of *Thujopsis dolabrata* may be a method of immersing 100 g of a dry pulverization product of *Thujopsis dolabrata* in 1 liter of ethanol, performing extraction for 7 days while intermittently stirring at room temperature, filtering an extract thus obtained, leaving the filtrate to stand for 3 days at 5°C, and then filtering the filtrate again to obtain a supernatant. Subsequently, a residue obtained by distilling off the solvent from the extract thus obtained, is dissolved in a solvent such as methanol, ethanol or ethyl acetate as appropriate; the solution is subjected to column chromatography using, for example, a hydrophilic polymer such as AMBERLITE XAD-2, DIAION HP-20 or TSK GEL HW-40; Sephadex such as SEPHADEX LH-20; a reversed-phase system silica gel or a silica gel; or a cellulose as a carrier, and using, for example, water, methanol, ethanol, ethyl acetate, chloroform, dichloromethane, hexane, acetone or benzene as an eluenting solvent; and the resultant is fractionated by, for example, thin layer chromatography while identifying the intended component. Thus, an intended substance can be obtained. Furthermore, optionally, the intended substance may also be purified by recrystallization using an appropriate solvent such as benzene, ether, hexane, acetone, methanol, ethanol or water.

Furthermore, the lignan of formula (I) can be synthesized by the methods described in the literature (for example, JP10-175861 A, Natural Product Report, 183-205, 1995 and the like), and the source is not particularly limited. Alternatively, the lignan of formula (I) can be purchased from Quality Phytochemicals, 2Daybiochem, BOC Sciences, or Jamson PharmaChem Technology, for example.

As disclosed in the Examples described below, the lignan of formula (I) has an action of significantly promoting cellular responsiveness to CGRP. Therefore, the lignan is useful as a cellular CGRP-responsiveness promoter. Furthermore, the lignan can exhibit effects such as improvement of skin circulation and promotion of dental pulp formation, through the CGRP-responsiveness promoting action, and as a result, the lignan is useful for the improvement of skin metabolism, promotion of blood circulation, promotion of blood flow, promotion of wound healing, or the prevention and/or amelioration of diseases such as hypertension, cardiac failure, hepatic disorder caused by ischemic reperfusion, renal failure, gastrointestinal ulceration, septicemia, osteoporosis, arrhythmia, subarachnoid hemorrhage, pulmonary hypertension, delayed type hypersensitivity, periodontal disease, gestational toxicosis and premature labor.

That is, the lignan of formula (I) can be used for the promotion of cellular responsiveness to CGRP. Alternatively, the lignan of formula (I) can be used for the improvement of skin circulation or the improvement of skin metabolism, for the promotion of blood circulation or blood flow, or for the promotion of wound healing. Again, alternatively, the lignan of formula (I) can be used for the prevention and/or amelioration of diseases such as hypertension, cardiac failure, hepatic disorder caused by ischemic reperfusion, renal failure, gastrointestinal ulceration, septicemia, osteoporosis, arrhythmia, subarachnoid hemorrhage, pulmonary hypertension, delayed type hypersensitivity, periodontal diseases, gestational toxicosis and premature labor. The relevant use may be a use in a human being or a non-human animal, or an in vitro use in, for example, a tissue, organ, cell and the like originating from the human being or the non-human animal.

Furthermore, the use of the lignan of formula (I) according to the present invention may be a therapeutic use, or may be a non-therapeutic use. In the present specification, the non-therapeutic use of the lignan of formula (I) may be, for example, a use of the lignan of formula (I), not as a medical practice, but for the improvement of skin circulation, promotion of blood circulation or blood flow or improvement of skin metabolism for the purpose of maintaining or promoting health, or for the improvement of skin metabolism or promotion of wound healing for cosmetic or esthetic purposes. Furthermore, for example, the non-therapeutic use of the lignan of formula (I) may be to provide the lignan of formula (I) with the intention of using the lignan of formula (I), not as a medical practice, but in order to obtain effects such as the improvement of skin circulation, promotion of blood circulation or blood flow, improvement of skin metabolism and promotion of wound healing, for the purpose of maintaining or promoting health or for cosmetic or esthetic purposes, while indicating such effects.

Therefore, an embodiment of the present invention provides a cellular CGRP-responsiveness promoter comprising the lignan of formula (I) as an active ingredient. The CGRP-responsiveness promoter can be used for the promotion of cellular responsiveness to CGRP, and based on the CGRP-responsiveness promoting action, the CGRP-responsiveness promoter can be used for the improvement of skin circulation or the improvement of skin metabolism, for the promotion of blood circulation or blood flow, for the promotion of wound healing, or for the prevention and/or amelioration of diseases such as hypertension, cardiac failure, hepatic disorder caused by ischemic reperfusion, renal failure, gastrointestinal ulceration, septicemia, osteoporosis, arrhythmia, subarachnoid hemorrhage, pulmonary hypertension, delayed type hypersensitivity, periodontal diseases, gestational toxicosis and premature labor. According to an embodiment of the present invention, the CGRP-responsiveness promoter essentially consists of the lignan of formula (I). Preferably, the CGRP-responsiveness promoter is used for the improvement of skin circulation or the improvement of skin metabolism, for the promotion of blood circulation or blood flow or for wound healing.

Another embodiment of the present invention provides a skin circulation improver comprising the lignan of formula (I) as an active ingredient. Still another embodiment of the present invention provides a skin metabolism improver comprising the lignan of formula (I) as an active ingredient. Preferably, the skin metabolism improver is an agent for ameliorating the reduction of skin metabolism caused by, for example, aging. Such an agent according to an exemplary embodiment essentially consists of the lignan of formula (I).

Furthermore, another embodiment of the present invention provides a blood circulation or blood flow promoter comprising the lignan of formula (I) as an active ingredient. Furthermore, another embodiment of the present invention provides a wound healing promoter comprising the lignan of formula (I) as an active ingredient. Furthermore, still another embodiment of the present invention provides an agent for the prevention and/or amelioration of diseases such as hypertension, cardiac failure, hepatic disorder caused by ischemic reperfusion, renal failure, gastrointestinal ulceration, septicemia, osteoporosis, arrhythmia, subarachnoid hemorrhage, pulmonary hypertension, delayed type hypersensitivity, periodontal diseases, gestational toxicosis and premature labor, the agent comprising the lignan of formula (I) as an active ingredient. According to an exemplary embodiment, such an agent essentially consists of the lignan of formula (I).

Furthermore, another embodiment of the present invention provides a use of the lignan of formula (I) for the production of a cellular CGRP-responsiveness promoter. Still another embodiment of the present invention provides a use of the lignan of formula (I) for the production of a skin circulation improver, a blood circulation promoter, a blood flow promoter, a wound healing promoter, or an agent for the prevention and/or amelioration of diseases such as hypertension, cardiac failure, hepatic disorder caused by ischemic reperfusion, renal failure, gastrointestinal ulceration, septicemia, osteoporosis, arrhythmia, subarachnoid hemorrhage, pulmonary hypertension, delayed type hypersensitivity, periodontal diseases, gestational toxicosis and premature labor. Preferably, the relevant skin metabolism improver is an agent for improving the reduction of skin metabolism caused by, for example, aging. According to an exemplary embodiment, the action of improving skin circulation, the action of promoting blood circulation or blood flow, the action of promoting wound healing, or the action of preventing and/or ameliorating the aforementioned diseases of the agent described above is caused by the action of promoting cellular responsiveness to CGRP.

The lignan of formula (I) can be used for the production of, for example, a composition, a medicine, a quasi-drug, a cosmetic product, a food and beverage product and the like, which are intended for the promotion of cellular responsiveness to CGRP, for the improvement of skin circulation or the improvement of skin metabolism, for the promotion of blood circulation or blood flow, for the promotion of wound healing, or for the prevention and/or amelioration of diseases such as hypertension, cardiac failure, hepatic disorder caused by ischemic reperfusion, renal failure, gastrointestinal ulceration, septicemia, osteoporosis, arrhythmia, subarachnoid hemorrhage, pulmonary hypertension, delayed type hypersensitivity, periodontal diseases, gestational toxicosis and premature labor. The relevant composition, medicine, quasi-drug, cosmetic product, and food and beverage product, for example, are also included in the scope of the present invention.

The composition, medicine, quasi-drug, cosmetic product, and food and beverage product, for example, can be produced or used for human being or non-human animal. The lignan of formula (I) may be incorporated into, for example, the composition, medicine, quasi-drug, cosmetic product, food and beverage product or the like, and may serve as an active ingredient for the promotion of cellular responsiveness to CGRP, for the improvement of skin circulation or the improvement of skin metabolism, for the promotion of blood circulation or blood flow, for the promotion of wound healing, or for the prevention and/or amelioration of diseases such as hypertension, cardiac failure, hepatic disorder caused by ischemic reperfusion, renal failure, gastrointestinal ulceration, septicemia, osteoporosis, arrhythmia, subarachnoid hemorrhage, pulmonary hypertension, delayed type hypersensitivity, periodontal diseases, gestational toxicosis and premature labor.

The medicine or quasi-drug contains the lignan of formula (I) as an active ingredient. The medicine or quasi-drug can be administered in any form of administration. The administration may be carried out orally or parenterally. Examples of the dosage form for oral administration include solid forms of administration such as a tablet, a coated tablet, a granule, a powder and a capsule; and liquid forms of administration such as an elixir, a syrup and a suspension. Examples of the dosage formforparenteral administration include an injection, an infusion, a transdermal preparation, a transmucosal preparation, a transnasal preparation, an enteral preparation, an inhalant, a suppository, a bolus and a patch.

The medicine or quasi-drug may contain the lignan of formula (I) alone, or may contain the lignan of formula (I) in combination with a pharmaceutically acceptable carrier. Examples of such a carrier include an excipient, a film-forming agent, a binder, a bulking agent, a disintegrant, a lubricating agent, a diluent, an osmotic pressure regulator, a pH adjusting agent, adispersant, an emulsifier, an antiseptic, a stabilizer, an antioxidat, a colorant, an ultraviolet absorber, a moisture retaining agent, a thickener, an activity enhancer, an anti-inflammatory agent, a disinfectant, a fragrance, a flavoring agent and an odorizing agent. Furthermore, the relevant medicine or quasi-drug may contain other active ingredients or pharmacological components as long as the CGRP-responsiveness promoting action of the lignan of formula (I) is not lost.

The medicine or quasi-drug can be produced from the lignan of formula (I), or by combining the lignan of formula (I) with the carrier described above and/or another active ingredient or pharmacological component as necessary, through a conventional method. The content of the lignan of formula (T) in the medicine or quasi-drug is preferably adjusted to 0.001% to 100% by mass, more preferably adjusted to 0.002% to 100% by mass, even more preferably adjusted to 0.005% to 100% by mass, even more preferably adjusted to 0.01% to 100% by mass, even more preferably adjusted to 0.05% to 100% by mass.

The food and beverage product contains the lignan of formula (I) as an active ingredient. The food and beverage product may be developed based on the concepts of, for example, promotion of the responsiveness to CGRP, improvement of skin circulation, promotion of blood circulation or blood flow, improvement of skin metabolism, wound healing and the like, and can be produced as a food and beverage product, a functional food and beverage product, a health food, a food and beverage product for patients or a food and beverage product for a specific health use, with the concept labeled on the product as needed. These food and beverage products can be distinguished from general food and beverage products by the labeling.

Examples of the form of the food and beverage product include various food products such as breads, cakes, noodles, confectionaries, jellies, frozen foods, ice creams, dairy products, soups, edible oils and seasonings; as well as forms the same as the above-described preparations for oral administration (for example, tablets, capsules, granules, powders and syrups), feed for livestock, and pet foods. Examples of the form of beverage include fruit juices, carbonated drinks, teabeverages, nearwater, sports drinks, milkbeverages, alcohol beverages, and soft drinks. The beverages can be prepared as packaged beverages filled in packaging containers.

In order to prepare the food and beverage products of various forms as described above, the lignan of formula (I) can be used alone, or the lignan of formula (I) can be used in appropriate combination with, for example, other food and beverage materials, a solvent, a softening agent, an oil, an emulsifier, anantiseptic, a fragrance, astabilizer, a colorant, an antioxidant, a moisturizer, a thickener and the like. The content of the lignan of formula (I), for example, in the food and beverage products is preferably adjusted to 0.00001% to 100% by mass, more preferably adjusted to 0.00002% to 75% by mass, even more preferably adjusted to 0.00005% to 50% by mass, even more preferably adjusted to 0.0001% to 30% by mass, even more preferably adjusted to 0.0002% to 10% by mass.

The cosmetic product contains the lignan of formula (I) as an active ingredient. The cosmetic product may contain the lignan of formula (I) alone, or may contain the lignan of formula (I) in combination with a cosmetically acceptable carrier. Examples of such a carrier include an excipient, a film-forming agent, a binder, a bulking agent, a disintegrant, a lubricating agent, a diluent, an osmotic pressure regulator, a pH adjusting agent, a dispersant, an emulsifier, an antiseptic agent, a stabilizer, an antioxidant, a colorant, an ultraviolet absorber, a thickener, an activity enhancer, an anti-inflammatory agent, a disinfectant, a fragrance, a flavoring agent and an odorizing agent. The cosmetic product may also contain other active ingredients or cosmetic components, for example, a moisturizer, a skin lightening agent, an ultraviolet screening agent, a cell activator, a detergent, a keratolytic agent, and makeup components (for example, makeup base, foundation, face powder, body powder, blusher, lipstick, eye shadow, eyebrow pencil, mascara and the like), as long as the CGRP-responsiveness promoting action of the lignan of formula (I) is not lost. Examples of the form of cosmetic product include any forms that may be used in cosmetic products, such as a cream, an emulsion, a lotion, a suspension, a gel, a powder, a pack, a sheet, a patch, a stick and a cake.

The cosmetic product can be produced from the lignan of formula (I), or by combining the lignan of formula (I) with the carrier described above and/or other active ingredients or cosmetic components as necessary, through a conventional method. The content of the lignan of formula (I) in the cosmetic product is preferably adjusted to 0.00001% to 100% by mass, more preferably adjusted to 0.00002% to 75% by mass, even more preferably adjusted to 0.00005% to 50% by mass, even more preferably adjusted to 0.0001% to 30% by mass, even more preferably adjusted to 0.0002% to 10% by mass.

Alternatively, the lignan of formula (I) may be administered in an effective amount to an animal in need of or desired for the promotion of responsiveness to CGRP, the improvement of skin circulation or the improvement of skin metabolism, the promotion of blood circulation or blood flow, the promotion of wound healing, or the prevention and/or amelioration of diseases such as hypertension, cardiac failure, hepatic disorder caused by ischemic reperfusion, renal failure, gastrointestinal ulceration, septicemia, osteoporosis, arrhythmia, subarachnoid hemorrhage, pulmonary hypertension, delayed type hypersensitivity, periodontal diseases, gestational toxicosis and premature labor. According to an embodiment, the administration is carried out non-therapeutically with intension of for example, improving skin circulation, improving skin metabolism, promoting blood circulation or blood flow, promoting wound healing or the like for the purpose of maintaining or promoting health or for a cosmetic or esthetic purpose.

The subject to be administered may be an animal in need of or desired for the improvement of skin circulation or the improvement of skin metabolism, and preferably an animal in need of or desired for the effect of an amelioration of the reduction of skin metabolism caused by, for example, aging. On the other hand, examples of the subject to be administered include an animal in need of or desired for the promotion of blood circulation or the promotion of wound healing, an animal in need of or desired for the promotion of dental pulp formation, and an animal suffering from, an animal suspected to have or an animal having a risk of, a disease such as hypertension, cardiac failure, hepatic disorder caused by ischemic reperfusion, renal failure, gastrointestinal ulceration, septicemia, osteoporosis, arrhythmia, subarachnoid hemorrhage, pulmonary hypertension, delayed type hypersensitivity, periodontal diseases, gestational toxicosis or premature labor. A preferred example of the subject to be administered may be an animal in need of or desired for the improvement of skin circulation or the improvement of skin metabolism, the promotion of blood circulation or blood flow or wound healing. The animal is preferably a human being or a non-human mammal, more preferably a human being.

A preferred amount administered may vary depending on the species, body weight, gender, age and condition of the subject, or other factors. The dose, route and interval of administration, and the amount or interval of intake may be appropriately determined by a person having ordinary skill in the art. For example, in the case of orally administering to a human being, the amount administered is, in terms of the lignan of formula (I), preferably 1 µg/day to 6000 mg/day, more preferably 10 µg/day to 5000 mg/day, even more preferably 100 µg/day to 4000 mg/day, even more preferably 500 µg/day to 3000 mg/day, for an adult. Alternatively, in the case of transdermally administering to a human being, the amount administered is, in terms of the lignan of formula (I), preferably 0.001 µg/day to 6000 mg/day, more preferably 0.002 µg/day to 5000 mg/day, even more preferably 0.005 µg/day to 4000 mg/day, even more preferably 0.01 µg/day to 3000 mg/day, for an adult.

The subject to be administered may be a tissue, an organ or a cell derived from an animal, or a culture product or fraction thereof. The tissue, organ, cell or a fraction thereof is a naturally occurring, biologically modified, or bioengineered tissue, organ, cell or a fraction thereof, each of which expresses a CGRP receptor or has a CGRP receptor. Therefore, the present invention also provides a method for promoting the responsiveness to CGRP in the tissue, organ or cell, or a culture product or fraction thereof. The present method includes, for example, a step of culturing a cell which has a CGRP receptor and for which it is desired to promote the responsiveness to CGRP, in the presence of the lignan of formula (I). When the cell is a cell culture product, the concentration of the lignan of formula (I) to be added is 0.00001 µM to 1000 µM, preferably 0.00002 µM to 500 µM, and more preferably 0.00005 µM to 200 µM. The timing for adding the lignan of formula (I) may be before CGRP simulation, and/or at the time of CGRP stimulation.

The present invention also includes the following use or method as exemplary embodiments. However, the present invention is not intended to be limited to these exemplary embodiments.
(1) A cellular CGRP-responsiveness promoter comprising, as an active ingredient, a lignan represented by the above formula (I), wherein R₁ represents a hydrogen atom or an alkoxy group; and R₂, R₃ and R₄, which may be identical or different, each represent an alkoxy group.
(2) The promoter as described in item (1), wherein R₁ is preferably a hydrogen atom or an alkoxy group having 1 to 3 carbon atoms; and R₂, R₃ and R₄ are each preferably a methoxy group.
(3) The promoter as described in item (1) or (2), wherein the cell is preferably a vascular smooth muscle cell.
(4) A skin circulation improver comprising, as an active ingredient, a compound represented by the above formula (I), wherein R₁ represents a hydrogen atom or an alkoxy group; and R₂, R₃ and R₄, which may be identical or different, each represent an alkoxy group.
(5) A blood circulation or blood flow promoter comprising, as an active ingredient, a compound represented by the above formula (I), wherein R₁ represents a hydrogen atom or an alkoxy group; and R₂, R₃ and R₄, which may be identical or different, each represent an alkoxy group.
(6) A skin metabolism improver comprising, as an active ingredient, a compound represented by the above formula (I), wherein R₁ represents a hydrogen atom or an alkoxy group; and R₂, R₃ and R₄, which may be identical or different, each represent an alkoxy group.
(7) A wound healing promoter comprising, as an active ingredient, a compound represented by the above formula (I), wherein R₁ represents a hydrogen atom or an alkoxy group; and R₂, R₃ and R₄, which may be identical or different, each represent an alkoxy group.
(8) The improver or promoter as described in any one of items (4) to (7), wherein R₁ is preferably a hydrogen atom or an alkoxy group having 1 to 3 carbon atoms; and R₂, R₃ and R₄ are each preferably a methoxy group.
(9) A method for promoting cellular responsiveness to CGRP, the method comprising a step of culturing a cell which has a CGRP receptor and for which it is desired to promote the responsiveness to CGRP, in the presence of the CGRP-responsiveness promoter as described in any one of items (1) to (3).
(10) The method as described in item (9), wherein the cell is preferably a vascular smooth muscle cell.
(11) Use of a lignan represented by the above formula (I), wherein R₁ represents a hydrogen atom or an alkoxy group; and R₂, R₃ and R₄, which may be identical or different, each represent an alkoxy group, for the promotion of cellular responsiveness to CGRP.
(12) The use described in item (11), wherein R₁ is preferably a hydrogen atom or an alkoxy group having 1 to 3 carbon atoms; and R₂, R₃ and R₄ are each preferably a methoxy group.
(13) The use described in item (11) or (12), wherein the cell is preferably a vascular smooth muscle cell.
(14) Use of a lignan represented by the above formula (I), wherein R₁ represents a hydrogen atom or an alkoxy group; and R₂, R₃ and R₄, which may be identical or different, each represent an alkoxy group, for the improvement of skin circulation.
(15) Use of a lignan represented by the above formula (I), wherein R₁ represents a hydrogen atom or an alkoxy group; and R₂, R₃ and R₄, which may be identical or different, each represent an alkoxy group, for the promotion of blood circulation or blood flow.
(16) Use of a lignan represented by the above formula (I), wherein R₁ represents a hydrogen atom or an alkoxy group; and R₂, R₃ and R₄, which may be identical or different, each represent an alkoxy group, for the improvement of skin metabolism.
(17) Use of a lignan represented by the above formula (I), wherein R₁ represents a hydrogen atom or an alkoxy group; and R₂, R₃ and R₄, which may be identical or different, each represent an alkoxy group, for the promotion of wound healing.
(18) The use as described in any one of items (14) to (17), wherein R₁ is preferably a hydrogen atom or an alkoxy group having 1 to 3 carbon atoms; and R₂, R₃ and R₄ are each preferably a methoxy group.
(19) The use as described in any one of items (11) to (18), wherein the use is preferably non-therapeutic use.
(20) The use as described in any one of items (11) to (19), wherein the lignan represented by the above formula (I) is preferably used in oral administration to a human being in an amount administered of 1 µg/day to 6000 mg/day for an adult.
(21) Use of a lignan represented by the above formula (I), wherein R₁ represents a hydrogen atom or an alkoxy group; and R₂, R₃ and R₄, which may be identical or different, each represent an alkoxy group, for the production of a cellular CGRP-responsiveness promoter.
(22) The use as described in item (21), wherein R₁ is preferably a hydrogen atom or an alkoxy group having 1 to 3 carbon atoms; and R₂, R₃ and R₄ are each preferably a methoxy group.
(23) The use as described in item (21) or (22), wherein the cell is preferably a vascular smooth muscle cell.
(24) Use of a lignan represented by the above formula (I), wherein R₁ represents a hydrogen atom or an alkoxy group; and R₂, R₃ and R₄, which may be identical or different, each represent an alkoxy group, for the production of a skin circulation improver.
(25) Use of a lignan represented by the above formula (I), wherein R₁ represents a hydrogen atom or an alkoxy group; and R₂, R₃ and R₄, which may be identical or different, each represent an alkoxy group, for the production of a blood circulation or blood flow promoter.
(26) Use of a lignan represented by the above formula (I), wherein R₁ represents a hydrogen atom or an alkoxy group; and R₂, R₃ and R₄, which may be identical or different, each represent an alkoxy group, for the production of a skin metabolism improver.
(27) Use of a lignan represented by the above formula (I), wherein R₁ represents a hydrogen atom or an alkoxy group; and R₂, R₃ and R₄, which may be identical or different, each represent an alkoxy group, for the production of a wound healing promoter.
(28) The use as described in any one of items (24) to (27), wherein R₁ is preferably a hydrogen atom or an alkoxy group having 1 to 3 carbon atoms; and R₂, R₃ and R₄ are each preferably a methoxy group.
(29) Use of a lignan represented by the above formula (I), wherein R₁ represents a hydrogen atom or an alkoxy group; and R₂, R₃ and R₄, which may be identical or different, each represent an alkoxy group, for the production of a functional food or health food for the promotion of cellular responsiveness to CGRP, improvement of skin circulation, promotion of blood circulation or blood flow, improvement of skin metabolism, or promotion of wound healing.
(30) The use as described in any one of items (21) to (29), wherein the promoter, improver or food is preferably orally administered to a human being in an amount administered of 1 µg/day to 6000 mg/day, in terms of the lignan represented by the above formula (I), for an adult.
(31) A method for promoting CGRP-responsiveness in cells of a human being or non-human mammal; the method comprising administering a lignan represented by the above formula (I), wherein R₁ represents a hydrogen atom or an alkoxy group; and R₂, R₃ and R₄, which may be identical or different, each represent an alkoxy group, to a human being or non-human mammal in need of or desired for the promotion of the responsiveness to CGRP.
(32) The method as described in item (31), wherein R₁ is preferably a hydrogen atom or an alkoxy group having 1 to 3 carbon atoms; and R₂, R₃ and R₄ are each preferably a methoxy group.
(33) The method as described in item (31) or (32), wherein the cell is a vascular smooth muscle cell.
(34) A method for improving skin circulation in a human or non-human mammal; the method comprising administering a lignan represented by the above formula (I), wherein R₁ represents a hydrogen atom or an alkoxy group; and R₂, R₃ and R₄, which may be identical or different, each represent an alkoxy group, to a human being or non-human mammal in need of or desired for the improvement of skin circulation.
(35) A method for promoting blood circulation or blood flow in a human being or non-human mammal; the method comprising administering a lignan represented by the above formula (I), wherein R₁ represents a hydrogen atom or an alkoxy group; and R₂, R₃ and R₄, which may be identical or different, each represent an alkoxy group, to a human being or non-human mammal in need of or desired for the promotion of blood circulation or blood flow.
(36) A method for improving skin metabolism in a human being or non-human mammal; the method comprising administering a lignan represented by the above formula (I), wherein R₁ represents a hydrogen atom or an alkoxy group; and R₂, R₃ and R₄, which may be identical or different, each represent an alkoxy group, to a human being or non-human mammal in need of or desired for the improvement of skin metabolism.
(37) A method for promoting wound healing in a human being or non-human mammal; the method comprising administering a lignan represented by the above formula (I), wherein R₁ represents a hydrogen atom or an alkoxy group; and R₂, R₃ and R₄, which may be identical or different, each represent an alkoxy group, to a human being or non-human mammal in need of or desired for the wound healing.
(38) The method as described in any one of items (34) to (37), wherein R₁ is preferably a hydrogen atom or an alkoxy group having 1 to 3 carbon atoms; and R₂, R₃ and R₄ are each preferably a methoxy group.
(39) The method as described in any one of items (31) to (38), wherein the method is preferably a non-therapeutic method.
(40) The method described in any one of items (31) to (39), wherein the lignan represented by the above formula (I) is preferably orally administered to a human being in an amount administered of 1 µg/day to 6000 mg/day for an adult.
(41) A lignan represented by the above formula (I), wherein R₁ represents a hydrogen atom or an alkoxy group; and R₂, R₃ and R₄, which may be identical or different, each represent an alkoxy group, for use in the promotion of cellular responsiveness to CGRP.
(42) The lignan as described in item (41), wherein R₁ is preferably a hydrogen atom or an alkoxy group having 1 to 3 carbon atoms; and R₂, R₃ and R₄ are each preferably a methoxy group.
(43) The lignan as described in item (41) or (42), wherein the cell is preferably a vascular smooth muscle cell.
(44) A lignan represented by the above formula (I), wherein R₁ represents a hydrogen atom or an alkoxy group; and R₂, R₃ and R₄, which may be identical or different, each represent an alkoxy group, for use in the improvement of skin circulation.
(45) A lignan represented by the above formula (I), wherein R₁ represents a hydrogen atom or an alkoxy group; and R₂, R₃ and R₄, which may be identical or different, each represent an alkoxy group, for use in the promotion of blood circulation or blood flow.
(46) A lignan represented by the above formula (I), wherein R₁ represents a hydrogen atom or an alkoxy group; and R₂, R₃ and R₄, which may be identical or different, each represent an alkoxy group, for use in the improvement of skin metabolism.
(47) A lignan represented by the above formula (I), wherein R₁ represents a hydrogen atom or an alkoxy group; and R₂, R₃ and R₄, which may be identical or different, each represent an alkoxy group, for use in the promotion of wound healing.
(48) The lignan as described in any one of items (44) to (47), wherein R₁ is preferably a hydrogen atom or an alkoxy group having 1 to 3 carbon atoms; and R₂, R₃ and R₄ are each preferably a methoxy group.
(49) The lignan as described in any one of items (41) to (48), wherein the lignan is preferably orally administered to a human being in an amount administered of 1 µg/day to 6000 mg/day for an adult.
(50) In regard to the items (1) to (49), the lignan represented by the above formula (I) is preferably any one or both of the compounds described below:

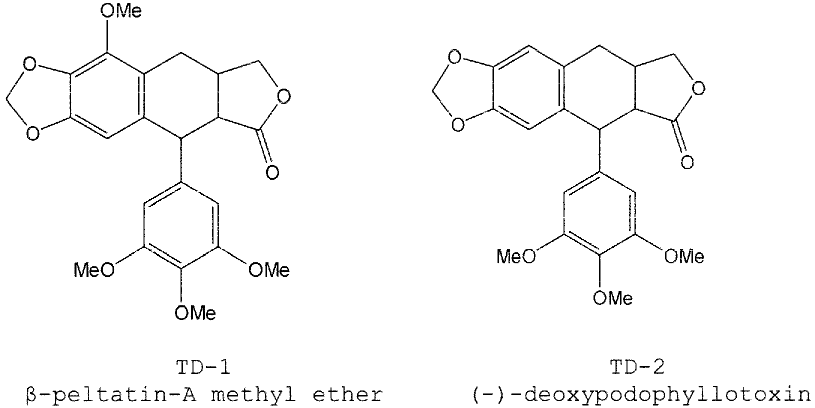

### Examples

Hereinafter, the present invention will be described more specifically by way of Examples.

### Example 1

The effect of a lignan on the promotion of cellular responsiveness to CGRP was investigated.

### 1. Method

### (1) Cell culture

Normal human coronary artery smooth muscle cells (HCASMC; Kurabo Industries, Ltd., cell lot #01272) were cultured in a growth medium (product obtained by adding 25 ml of FBS, 0.5 ml of hEGF, 0.5 ml of hFGF-B, 0.5 ml of insulin and 0.5 ml of an antibacterial agent to 500 ml of basal medium HuMedia-SB2; Kurabo Industries, Ltd). 0.025% Trypsin/0.01% EDTA was used for the passage.

### (2) Preparation of cells

Normal human coronary artery smooth muscle cells (HCASMC) were inoculated into a 48-well plate using the growth medium at a density of 4000 cells/cm², and the next day, the medium was replaced with a differentiation medium (product obtained by adding 5 ml of FBS, 0.5 ml of heparin and 0.5 ml of an antibacterial agent to 500 ml of basal medium HuMedia-SB2; Kurabo Industries, Ltd). The medium was replaced every two days, and the cells were cultured for 7 days, followed by the measurement of the responsiveness to CGRP.

### (3) Measurement of responsiveness to CGRP

The cells were washed twice with a basal medium (HuMedia-SB2; Kurabo Industries, Ltd.) containing phosphodiesterase inhibitors (PDI), IBMX (3-isobutyl-1-methylxanthine, 500 µM, Sigma-Aldrich Co.), Ro-20-1724 (100 µM; Wako Pure Chemical Industries, Ltd.), and a lignan compound shown below at a predetermined concentration. 200 µl of the same medium was further added to the cells, and the cells were incubated at 37°C for 60 minutes to allow the inhibitors to infiltrate into the cells. Themediumwas removed, a basal medium containing a lignan compound shown below at the same concentration, 0.5 nM CGRP and PDI was added to the cells, and the cells were incubated at 37°C for 15 minutes. Termination of the reaction was carried out by adding a lysis buffer attached to a cAMP analysis EIA kit (cAMP EIA (non-acetylation); GE Healthcare Biosciences Corp.) to the cells, and the intracellular cAMP concentration ([cAMP]i) was measured according to the method described in the manual. The CGRP responsiveness promotion capacity is provided by using the [cAMP]i obtained when stimulated with only 0.5 nM CGRP as a reference (control) and the percentage of the [cAMP] i is represented as an Improve Index.

### (4) Test compound

The following lignan compounds TD-1 and TD-2 were used for the test. These compounds were prepared by the following procedure, based on the method described in JP 10-175861 A. A dry pulverization product of *Thujopsis dolabrata* (180 g) was immersed in 1 L of ethanol, and extraction was carried out for 7 days at room temperature while the mixture was intermittently stirred. The extract thus obtained was filtered, the filtrate was left to stand for 3 days at 5°C, and then the filtrate was filtered again to obtain a supernatant. Next, a residue (2.4 g) obtained by distilling off the solvent was subjected to silica gel column chromatography (hexane:ethyl acetate, CHCl₃:CH₃OH, H₂O: CH₃OH) and high performance liquid chromatography (YMC-ODS, S-10, manufactured by YMC Co., Ltd.), and thus the compounds were prepared (yield: TD-1 21 mg, TD-2 25 mg).

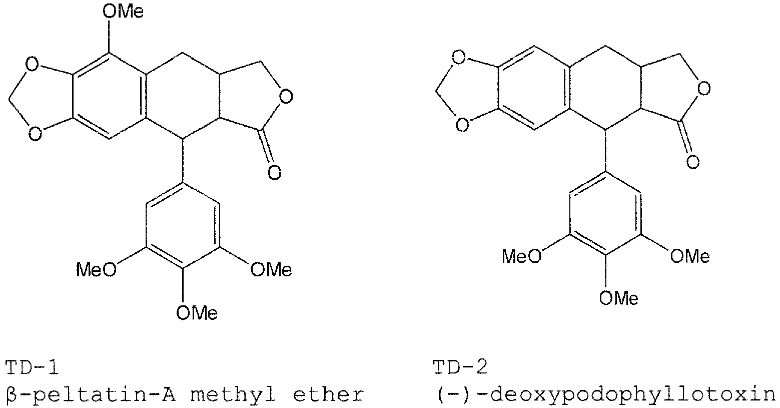

### 2. Results

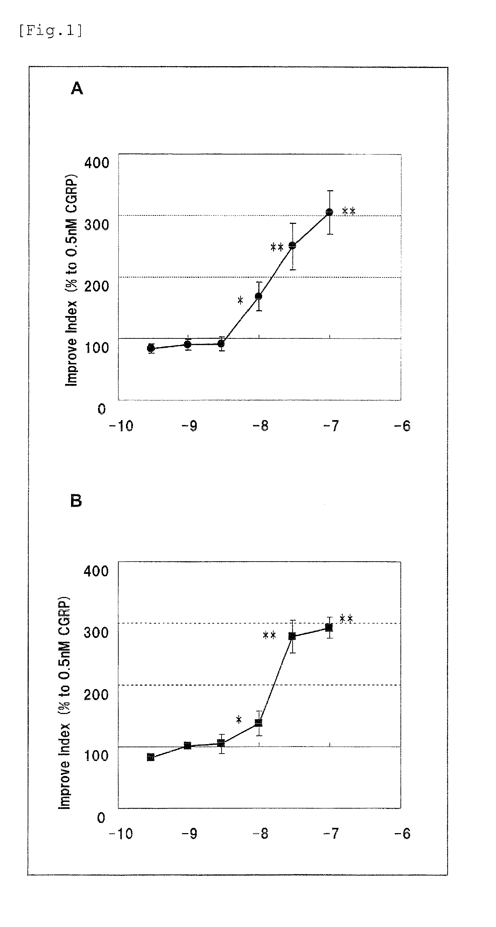

The results for the measurement of the responsiveness to CGRP are presented in Fig. 1, A and B. An Improve Index of 100% corresponds to 1189.3 fmol/well of [cAMP]i. The compounds TD-1 and TD-2 significantly promoted the cellular responsiveness to CGRP in a concentration-dependent manner (Dunnett's test, N = 3) .

### Example 2

The cellular responsiveness to CGRP was investigated by changing the conditions for the addition of compounds in various ways.

### 1. Method

HCASMC (Kurabo Industries, Ltd., cell lot #01272) was prepared under the same conditions as those used in Example 1. Thereafter, phosphodiesterase inhibitors (PDI), a test compound (TD-1 or TD-2, 100 nM), and/or CGRP (0.5 nM) were added to the culture product under the conditions described in Table 1, and the cells were incubated. Thereafter, the cellular responsiveness to CGRP was measured by the same method as that used in Example 1.

### 2. Results

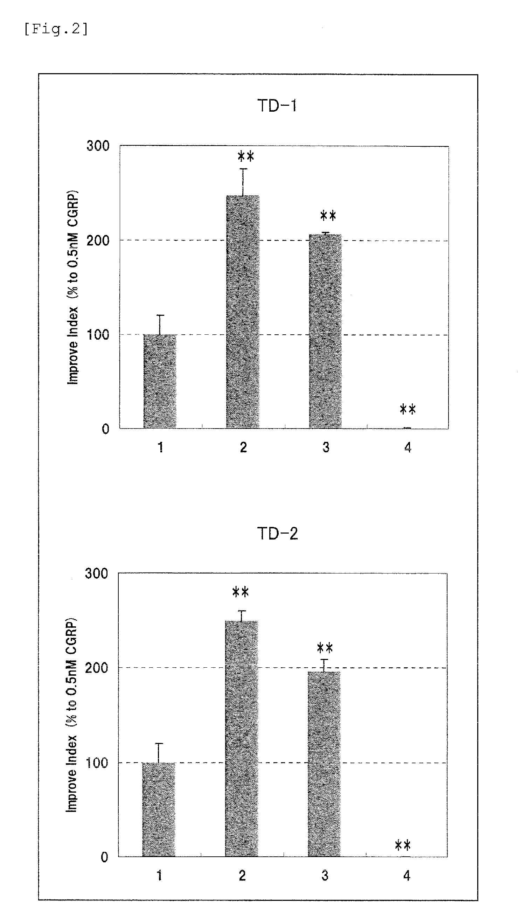

The results for the measurement of the responsiveness to CGRP are presented in Fig. 2. An Improve Index of 100% corresponds to 1189.3 fmol/well of [cAMP]i. When a test compound was added at the time of CGRP stimulation, the Improve Index increased (Conditions 2 and 3). On the other hand, the Improve Index was significantly low in the absence of CGRP (Condition 4). Therefore, it was found that the action brought by the test compound was not a compound substituting CGRP (for example, a CGRP receptor agonist), but was a compound promoting a series of responses induced by the binding between CGRP and a receptor thereof.

**[Table 1]**

| Conditions | Upon PDI infiltration | Upon CGRP stimulation | |
|---|---|---|---|
| | Test Compound | 0.5nM CGRP | Test compound |
| 1 | - | + | - |
| 2 | 100 nM | + | 100 nM |
| 3 | - | + | 100 nM |
| 4 | - | - | 100 nM |

## Claims

1. Use of a lignan represented by the following formula (I), for the production of a cellular CGRP-responsiveness promoter: 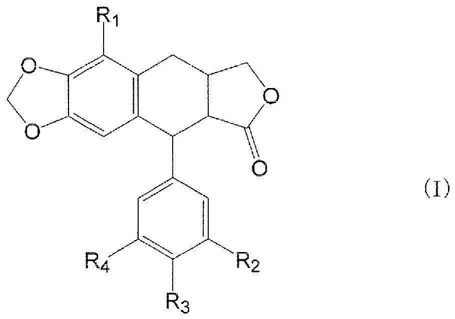 wherein R₁ represents a hydrogen atom or an alkoxy group; and R₂, R₃ and R₄, which may be identical or different, each represent an alkoxy group.

2. The use according to claim 1, wherein R₁ represents a hydrogen atom or an alkoxy group having 1 to 3 carbon atoms; and R₂, R₃ and R₄ each represent a methoxy group.

3. The use according to claim 1, wherein the lignan represented by the formula (I) is any one or both of the following compounds:

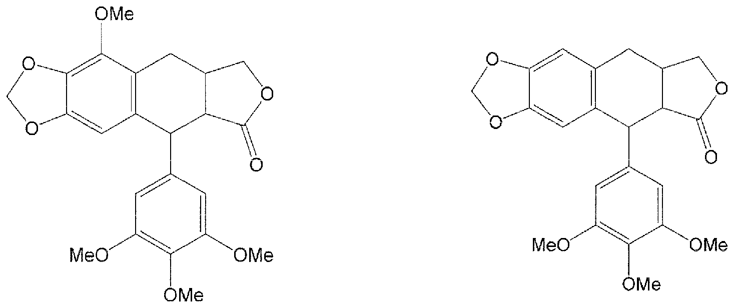

4. The use according to any one of claims 1 to 3, wherein the cell is a vascular smooth muscle cell.

5. Use of a lignan represented by the following formula (I), for the production of a skin circulation improver: 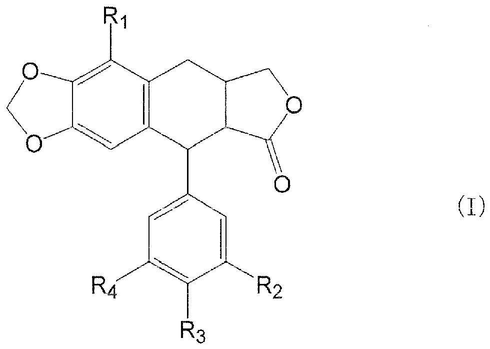 wherein R₁ represents a hydrogen atom or an alkoxy group; and R₂, R₃ and R₄, which may be identical or different, each represent an alkoxy group.

6. Use of a lignan represented by the following formula (I), for the production of a blood circulation or blood flow promoter : 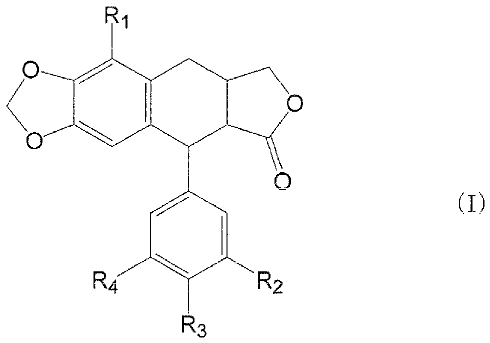 wherein R₁ represents a hydrogen atom or an alkoxy group; and R₂, R₃ and R₄, which may be identical or different, each represent an alkoxy group.

7. Use of a lignan represented by the following formula (I), for the production of a skin metabolism improver: 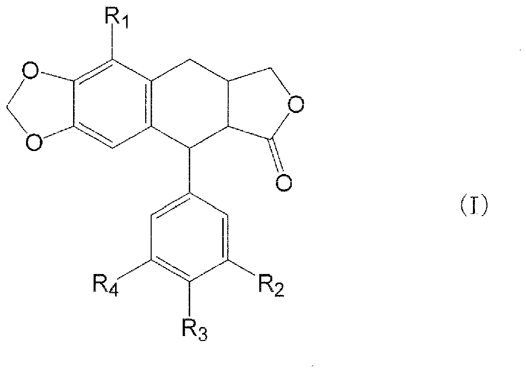 wherein R₁ represents a hydrogen atom or an alkoxy group; and R₂, R₃ and R₄, which may be identical or different, each represent an alkoxy group.

8. Use of a lignan represented by the following formula (I), for the production of a wound healing promoter: 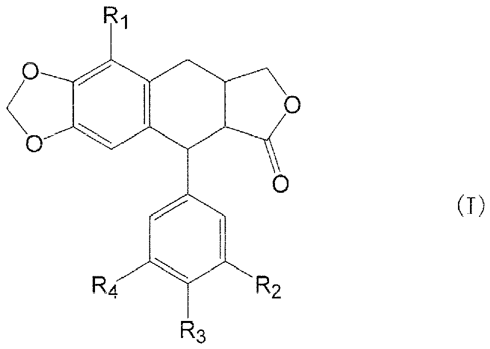 wherein R₁ represents a hydrogen atom or an alkoxy group; and R₂, R₃ and R₄, which may be identical or different, each represent an alkoxy group.

9. The use according to any one of claims 5 to 8, wherein R₁ represents a hydrogen atom or an alkoxy group having 1 to 3 carbon atoms; and R₂, R₃ and R₄ each represent a methoxy group.

10. The use according to any one of claims 5 to 8, wherein the lignan represented by the formula (I) is any one or both of the following compounds:

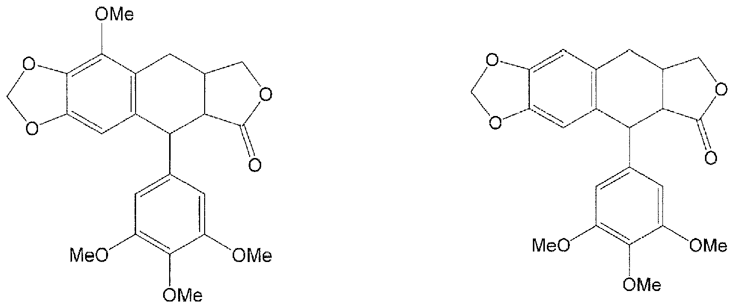

11. Non-therapeutic use of a lignan represented by the following formula (I), for the promotion of cellular responsiveness to CGRP: 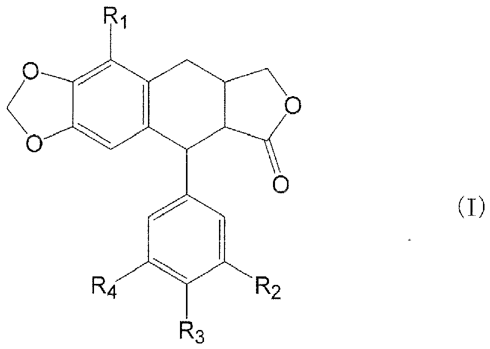 wherein R₁ represents a hydrogen atom or an alkoxy group; and R₂, R₃ and R₄, which may be identical or different, each represent an alkoxy group.

12. The use according to claim 11, wherein R₁ represents a hydrogen atom or an alkoxy group having 1 to 3 carbon atoms; and R₂, R₃ and R₄ each represent a methoxy group.

13. The use according to claim 11, wherein the lignan represented by the formula (I) is any one or both of the following compounds:

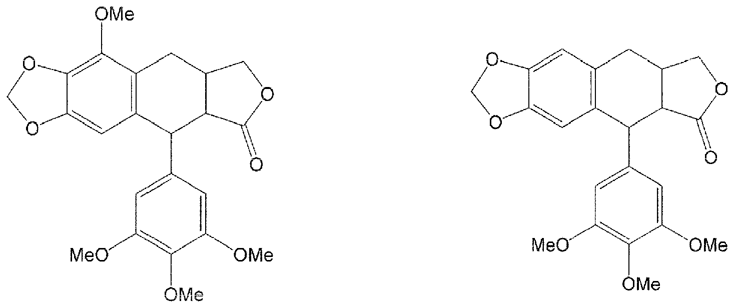

14. The use according to any one of claims 11 to 13, wherein the cell is a vascular smooth muscle cell.

15. Non-therapeutic use of a lignan represented by the following formula (I), for the improvement of skin circulation: 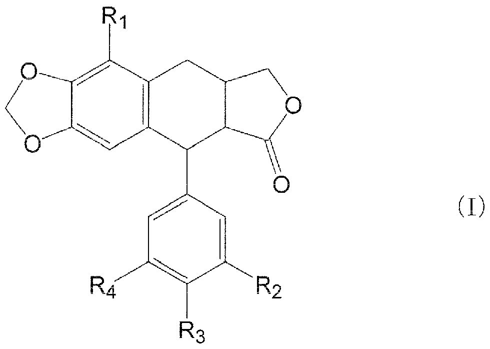 wherein R₁ represents a hydrogen atom or an alkoxy group; and R₂, R₃ and R₄, which may be identical or different, each represent an alkoxy group.

16. Non-therapeutic use of a lignan represented by the following formula (I), for the promotion of blood circulation or blood flow: 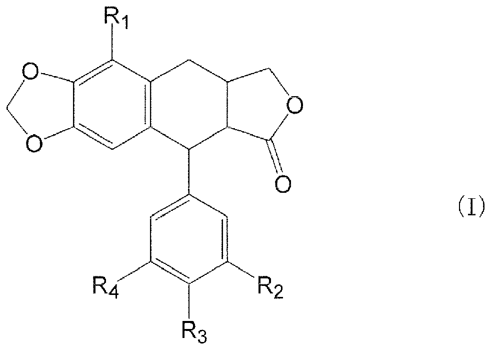 wherein R₁ represents a hydrogen atom or an alkoxy group; and R₂, R₃ and R₄, which may be identical or different, each represent an alkoxy group.

17. Non-therapeutic use of a lignan represented by the following formula (I), for the improvement of skin metabolism: 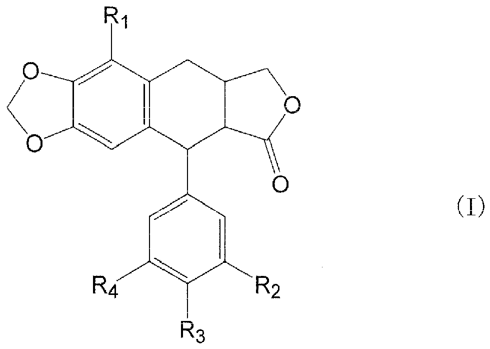 wherein R₁ represents a hydrogen atom or an alkoxy group; and R₂, R₃ and R₄, which may be identical or different, each represent an alkoxy group.

18. Non-therapeutic use of a lignan represented by the following formula (I), for the promotion of wound healing: 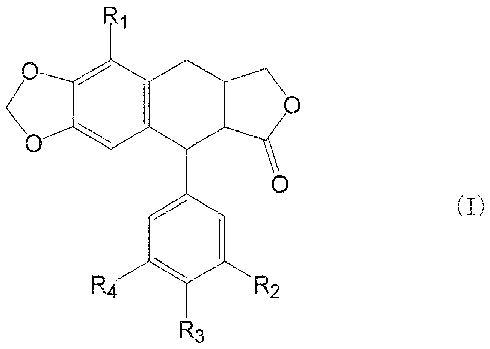 wherein R₁ represents a hydrogen atom or an alkoxy group; and R₂, R₃ and R₄, which may be identical or different, each represent an alkoxy group.

19. The use according to any one of claims 15 to 18, wherein R₁ represents a hydrogen atom or an alkoxy group having 1 to 3 carbon atoms; and R₂, R₃ and R₄ each represent a methoxy group.

20. The use according to any one of claims 15 to 18, wherein the lignan represented by the formula (I) is any one or both of the following compounds:

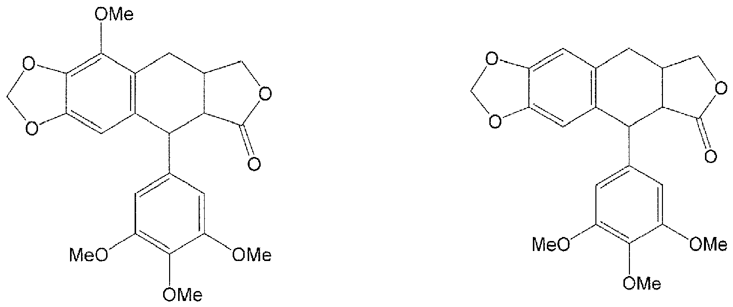

21. A lignan represented by the following formula (I), for use in the promotion of cellular responsiveness to CGRP: 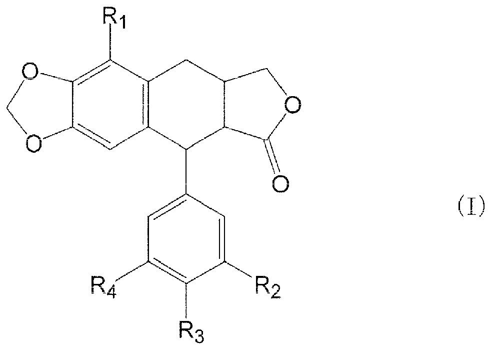 wherein R₁ represents a hydrogen atom or an alkoxy group; and R₂, R₃ and R₄, which may be identical or different, each represent an alkoxy group.

22. The lignan according to claim 21, wherein R₁ represents a hydrogen atom or an alkoxy group having 1 to 3 carbon atoms; and R₂, R₃ and R₄ each represent a methoxy group.

23. The lignan according to claim 21, wherein the lignan is any one or both of the following compounds:

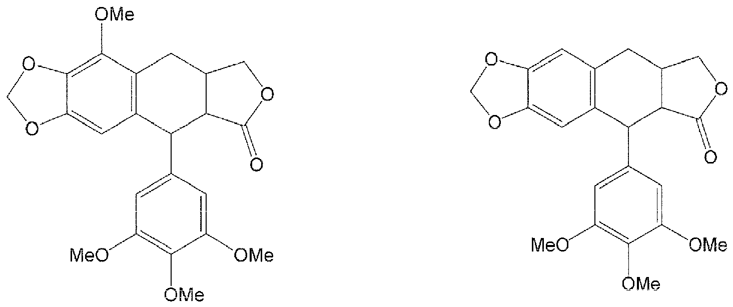

24. The lignan according to any one of claims 21 to 23, wherein the cell is a vascular smooth muscle cell.

25. A lignan represented by the following formula (I), for use in the improvement of skin circulation: 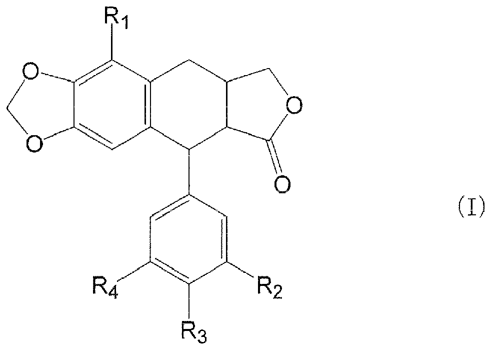 wherein R₁ represents a hydrogen atom or an alkoxy group; and R₂, R₃ and R₄, which may be identical or different, each represent an alkoxy group.

26. A lignan represented by the following formula (I), for use in the promotion of blood circulation or blood flow: 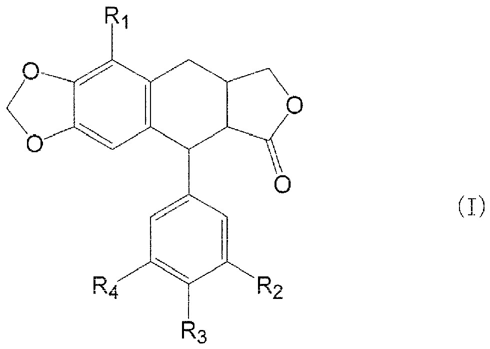 wherein R₁ represents a hydrogen atom or an alkoxy group; and R₂, R₃ and R₄, which may be identical or different, each represent an alkoxy group.

27. A lignan represented by the following formula (I), for use in the improvement of skin metabolism: 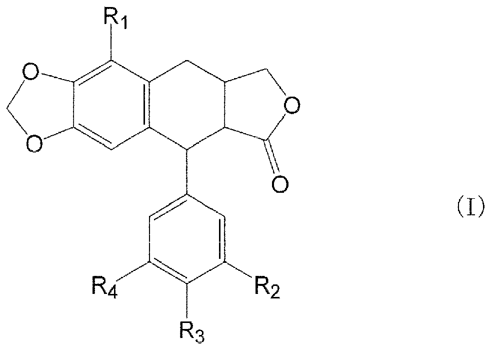 wherein R₁ represents a hydrogen atom or an alkoxy group; and R₂, R₃ and R₄, which may be identical or different, each represent an alkoxy group.

28. A lignan represented by the following formula (I), for use in the promotion of wound healing: 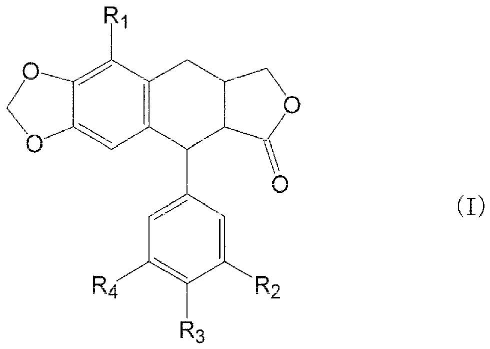 wherein R₁ represents a hydrogen atom or an alkoxy group; and R₂, R₃ and R₄, which may be identical or different, each represent an alkoxy group.

29. The lignan according to any one of claims 25 to 28, wherein R₁ represents a hydrogen atom or an alkoxy group having 1 to 3 carbon atoms; and R₂, R₃ and R₄ each represent a methoxy group.

30. The lignan according to any one of claims 25 to 28, wherein the lignan is any one or both of the following compounds:

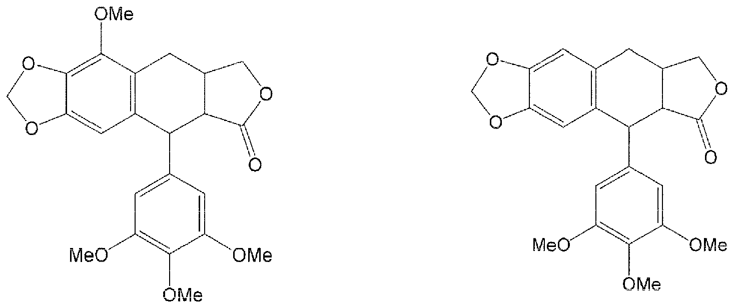

31. A method for promoting CGRP-responsiveness in cells of a human being or a non-human mammal, the method comprising administering a lignan represented by the following formula (I) to the human being or the non-human mammal: 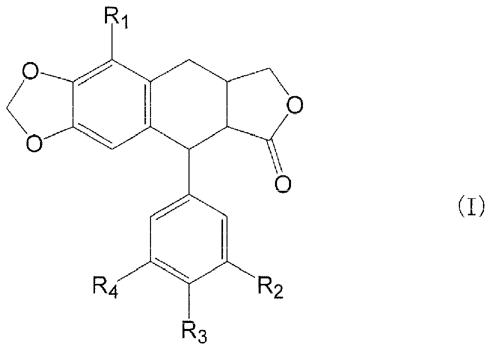 wherein R₁ represents a hydrogen atom or an alkoxy group; and R₂, R₃ and R₄, which may be identical or different, each represent an alkoxy group.

32. The method according to claim 31, wherein R₁ is preferably a hydrogen atom or an alkoxy group having 1 to 3 carbon atoms; and R₂, R₃ and R₄ are each preferably a methoxy group.

33. The method according to claim 31, wherein the lignan represented by the formula (I) is any one or both of the following compounds:

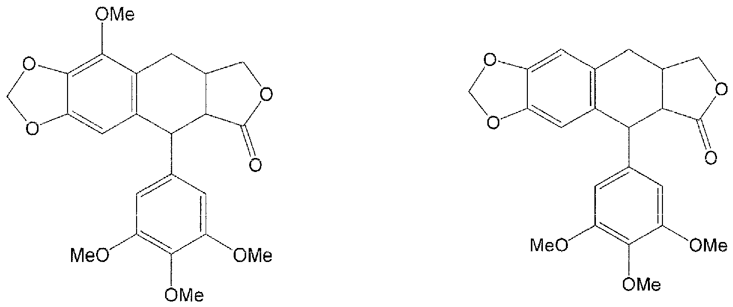

34. The method according to any one of claims 31 to 33, wherein the cell is preferably a vascular smooth muscle cell.

35. A method for improving skin circulation in a human being or a non-human mammal, the method comprising administering a lignan represented by the following formula (I) to the human being or the non-human mammal: 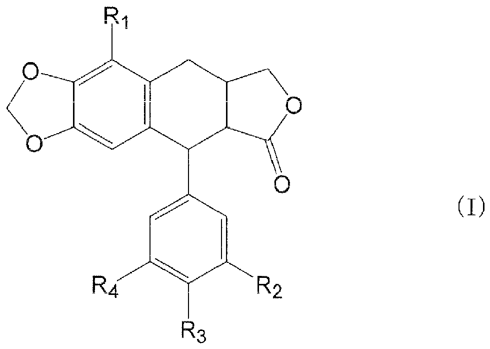 wherein R₁ represents a hydrogen atom or an alkoxy group; and R₂, R₃ and R₄, which may be identical or different, each represent an alkoxy group.

36. A method for promoting blood circulation or blood flow in a human being or a non-human mammal, the method comprising administering a lignan represented by the following formula (I) to the human being or the non-human mammal: 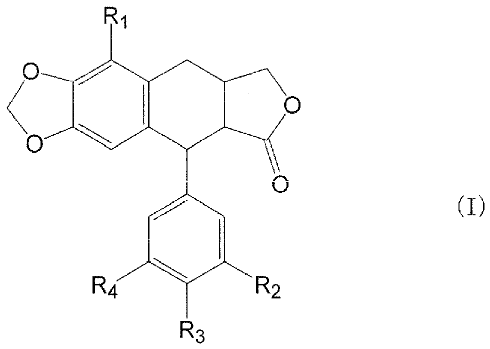 wherein R₁ represents a hydrogen atom or an alkoxy group; and R₂, R₃ and R₄, which may be identical or different, each represent an alkoxy group.

37. A method for improving skin metabolism in a human being or a non-human mammal, the method comprising administering a lignan represented by the following formula (I) to the human being or the non-human mammal: 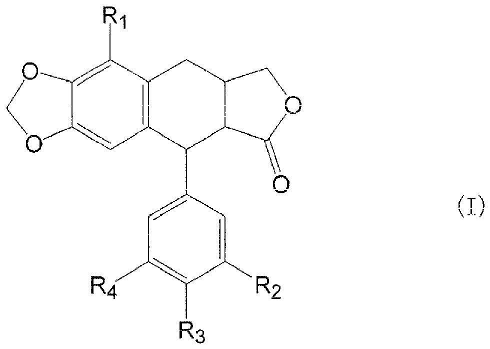 wherein R₁ represents a hydrogen atom or an alkoxy group; and R₂, R₃ and R₄, which may be identical or different, each represent an alkoxy group.

38. A method for promoting wound healing in a human being or a non-human mammal, the method comprising administering a lignan represented by the following formula (I) to a human being or a non-human mammal: 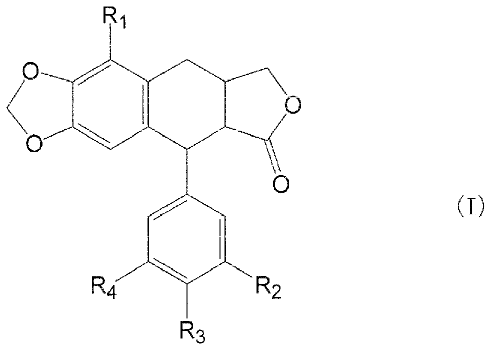 wherein R₁ represents a hydrogen atom or an alkoxy group; and R₂, R₃ and R₄, which may be identical or different, each represent an alkoxy group.

39. The method according to any one of claims 35 to 38, wherein R₁ represents a hydrogen atom or an alkoxy group having 1 to 3 carbon atoms; and R₂, R₃ and R₄ each represent a methoxy group.

40. The method according to any one of claims 35 to 38, wherein the lignan represented by the formula (I) is any one or both of the following compounds:

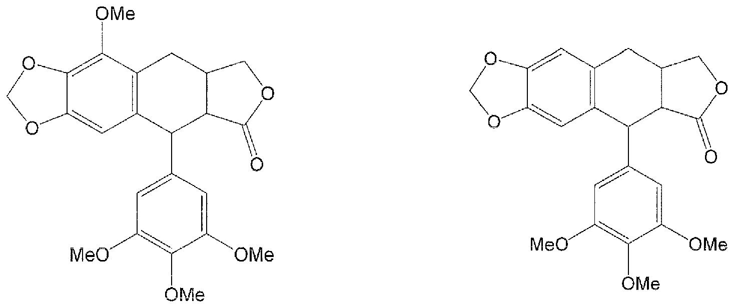

41. The method according to any one of claims 31 to 40, wherein the method is a non-therapeutic method.
